# EUROPEAN PATENT SPECIFICATION

(11) **EP 4 691 589 B1**
(45) Date of publication and mention of the grant of the patent: **02.09.2026**
(21) Application number: 24850249.4
(22) Date of filing: 11.07.2024
(51) Int. Cl.: B01D 3/00, C07C 29/80, C07C 31/10, B01D 3/32, B01D 3/14

(54) **REFINERY APPARATUS**
RAFFINERIEVORRICHTUNG
APPAREIL DE RAFFINAGE

(30) Priority: 19.09.2023 KR 20230124688; 09.07.2024 KR 20240090008
(43) Date of publication of application: 11.02.2026
(73) Proprietor: LG Chem, Ltd., Seoul 07336 (KR)
(72) Inventor: JANG, Kyung Soo, Daejeon 34122 (KR); LEE, Sung Kyu, Daejeon 34122 (KR); HWANG, Sung June, Daejeon 34122 (KR)
(74) Representative: Goddar, Heinz J.
(86) International application number: PCT/KR2024/009905
(87) International publication number: WO 2025/063453

(56) References cited:
- WO-A1-2018/080665
- CA-A1- 2 921 546
- CN-U- 206 809 811
- JP-A- 2022 516 377
- KR-A- 20140 003 337
- KR-A- 20230 039 362
- KR-B1- 102 414 715
- US-A1- 2018 361 270

## Description

### [TECHNICAL FIELD]

The present invention relates to a refinery apparatus, and more specifically, to a refinery apparatus capable of removing inorganic impurities from a feed stream including inorganic substances as impurities.

### [BACKGROUND]

Generally, in order to produce a compound, multiple substances are mixed and produced using multiple reactions. Unnecessary substances are generated in multiple reaction processes, which reduces the production efficiency of the product. In particular, when producing a compound using a reaction which uses a catalyst, inorganic impurities are included, which reduces the purity of the product. Therefore, in order to obtain a high-purity product, it is necessary to remove unnecessary impurities.

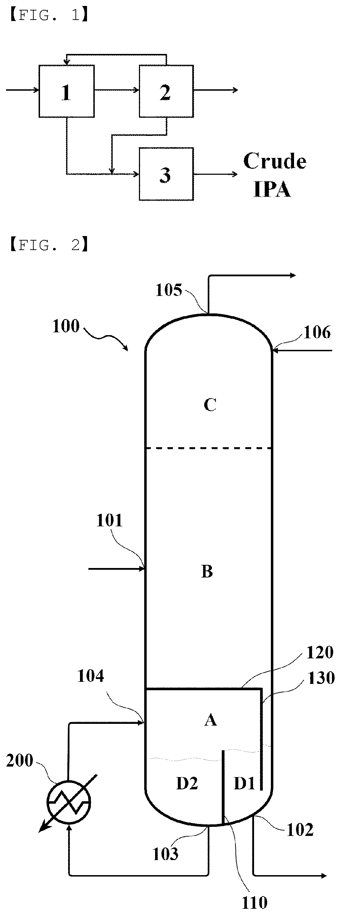

For example, in the case of producing isopropyl alcohol (IPA), referring to FIG. 1, typically, in the reaction unit 1, a propylene monomer and water are reacted to obtain a reaction product including isopropyl alcohol, unreacted propylene monomer, unreacted water, n-propyl alcohol (NPA), and by-products such as organic substances, the reaction product is transferred to a gas refinery unit 2 to separate low-boiling-point gas components including unreacted propylene monomer, and then the reaction product from which the gas components are separated is supplied to an IPA refinery unit 3 including a plurality of distillation columns to remove organic substances, NPA, and water, thereby obtaining crude isopropyl alcohol. Here, the crude isopropyl alcohol product obtained through the gas refinery unit and the IPA refinery unit may still include trace amounts of organic substances and water, and in particular, there is a problem of including inorganic impurity of a metal component due to the catalyst used in the reaction.

CN 206809811U discloses a refinery apparatus, comprising: a distillation column; and a reboiler, wherein an interior of the distillation column is divided into a bottom region, a middle region, and a top region, the bottom region comprises a first region and a second region separated by a partition wall extending from a bottom of the distillation column to have a predetermined height therebetween, the distillation column comprises: a feed supply port provided at one side of the middle region to supply a feed stream including a material to be refined to the distillation column, a first outlet provided below the first region, a second outlet provided below the second region and connected to one side of the reboiler, and a reflux inlet provided above the second region and connected to the other side of the reboiler, and a plurality of trays arranged vertically apart from the partition wall provided in the middle region of the distillation column, wherein one end of a lowest tray among the plurality of trays is opened so as not to contact a side of the distillation column, and a guide baffle extending downward from one end of the lowest tray to be adjacent to the bottom of the distillation column is provided.

### [SUMMARY]

### [Technical Problem]

The problem to be solved in the present invention is to solve the problem mentioned in the technology that is the background of the above invention, and an object of the present invention is to provide a refinery apparatus capable of obtaining a refined substance with high purity by effectively removing inorganic substances remaining in a feed stream including substances to be refined by using a refinery apparatus having an improved structure of a bottom region of a distillation column.

However, the problem to be solved by the present application is not limited to the problems mentioned above, and other problems that are not mentioned can be clearly understood by those skilled in the art from the description below.

### [Technical Solution]

According to one embodiment of the present invention, provided is a refinery apparatus comprising:
a distillation column; and
a reboiler,
wherein an interior of the distillation column is divided into a bottom region, a middle region, and a top region,
the bottom region comprises a first region and a second region separated by a partition wall extending from a bottom of the distillation column to have a predetermined height therebetween,
the distillation column comprises:
   a feed supply port provided at one side of the middle region to supply a feed stream including a material to be refined to the distillation column,
   a first outlet provided below the first region,
   a second outlet provided below the second region and connected to one side of the reboiler,
   a reflux inlet provided above the second region and connected to the other side of the reboiler, and
   a plurality of trays arranged vertically apart from the partition wall provided in the middle region of the distillation column,
   wherein one end of a lowest tray among the plurality of trays is opened so as not to contact a side of the distillation column on the first region, and a guide baffle extending downward from one end of the lowest tray (120) is provided,
   wherein a height h from the bottom of the distillation column to a lower end of the guide baffle satisfies the following Equation 1:
      $0,5 d < h < 2 d$
   wherein, d is a maximum gap opening between one end of the lowest tray and the side of the distillation column.

Further embodiments are disclosed in the dependent claims.

### [Advantageous Effects]

According to the present invention, by using a refinery apparatus that improves the structure of the bottom region of the distillation column, inorganic substances remaining in the material to be refined can be effectively removed, thereby achieving high purity refinery.

More specifically, the refinery apparatus according to the present invention can minimize the content of inorganic substances introduced into the reboiler by separating a feed inflow zone and a reboiler zone in the sump by providing a partition wall in the bottom region of the distillation column.

Furthermore, the refinery apparatus according to the present invention can maximize an inorganic substance removal rate by precipitating high-density inorganic substances in the material to be refined downward and then discharging precipitated inorganic substances downward by the guide baffle provided in the lowest tray and extended adjacent to the bottom of the distillation column.

The effects that can be obtained in the present invention are not limited to the effects mentioned above, and other effects that are not mentioned can be clearly understood by a person having ordinary knowledge in the technical field to which the present disclosure belongs from the description below.

### [DESCRIPTION OF THE DRAWINGS]

FIG. 1 is an example of material to be refined, and illustrates a typical process for manufacturing crude isopropyl alcohol.
FIG. 2 is a schematic diagram illustrating the overall structure of a refinery apparatus according to one embodiment of the present invention.
FIG. 3 is a structural diagram illustrating a bottom region of a distillation column in a refinery apparatus according to one embodiment of the present invention.
FIG. 4 is a structural diagram illustrating a bottom region of a distillation column in a refinery apparatus according to one embodiment of the present invention.
FIG. 5 is a structural diagram illustrating a bottom region of a distillation column in a refinery apparatus according to a comparative example.

### [DETAILED DESCRIPTION]

Terms or words used in the description and claims of the present disclosure should not be interpreted as limited to their usual or dictionary meanings, and should be interpreted as meanings and concepts that conform to the technical idea of the present disclosure, based on the principle that the inventor can appropriately define the concept of the term to best explain his or her invention.

In connection with the description of the drawings, similar reference symbols may be used for similar or related components.

The singular form of a noun corresponding to an item may include one or more of the items, unless the relevant context clearly indicates otherwise.

In the present disclosure, each of the phrases "A or B", "at least one of A and B", "at least one of A or B", "A, B or C", "at least one of A, B and C", and "at least one of A, B, or C" may include any one of the items listed together in the corresponding phrase, or all possible combinations thereof.

The term "and/or" includes a combination of multiple related described components or any one of multiple related described components.

Terms such as "first," "second," or "first" or "second" may be used simply to distinguish the corresponding component from other corresponding components and do not limit the corresponding components in other aspects (for example, importance or order).

In addition, terms such as "front", "back", "top", "bottom", "side", "left", "right", "upper", and "lower" used herein are defined based on the drawings, and the shape and location of each component are not limited by these terms.

Terms such as "include" or "have" are intended to specify the presence of a feature, number, step, operation, component, part, or combination thereof described in the present disclosure, but do not preclude the presence or addition of one or more other features, numbers, steps, operations, components, parts, or combinations thereof.

When a component is said to be "connected," "joined," "supported," or "in contact with" another component, this includes not only cases where the components are directly connected, joined, supported, or in contact, but also cases where they are indirectly connected, joined, supported, or in contact through a third component.

When a component is said to be "on" another component, this includes not only cases where the component is in contact with the other component, but also cases where another component exists between the two components.

In addition, the terms "about", "substantially", and the like used herein are used in the sense of the numerical value or a meaning close to the numerical value when the manufacturing and material tolerance inherent in the meaning mentioned are presented, and are used to prevent unscrupulous infringers from unfairly using the disclosure contents that mention exact or absolute values to help understand the present invention.

The term "stream" used herein may mean the flow of fluid within a process, and may also mean the fluid itself flowing within a pipe. Specifically, the stream may mean the fluid itself flowing within a pipe connecting each device, and the flow of the fluid at the same time. In addition, the fluid may include at least one component of gas, liquid, and solid.

In addition, the "pressure" mentioned herein means a gauge pressure measured based on atmospheric pressure.

The material to be refined mentioned herein may include a target product, a low-boiling-point organic substance, a high-boiling-point organic substance, and an inorganic substance, in which the low-boiling-point organic substance may mean an organic substance having a relatively low boiling point compared to the product, and the high-boiling-point organic substance may mean an organic substance having a relatively high boiling point compared to the product.

Hereinafter, the refinery apparatus of the present disclosure is described with reference to the attached drawings, but the attached drawings are exemplary and the scope of the refinery apparatus is not limited thereto. In addition, although isopropyl alcohol is described as an example of the target product below, the material to be refined of the refinery apparatus according to the present disclosure is not limited thereto.

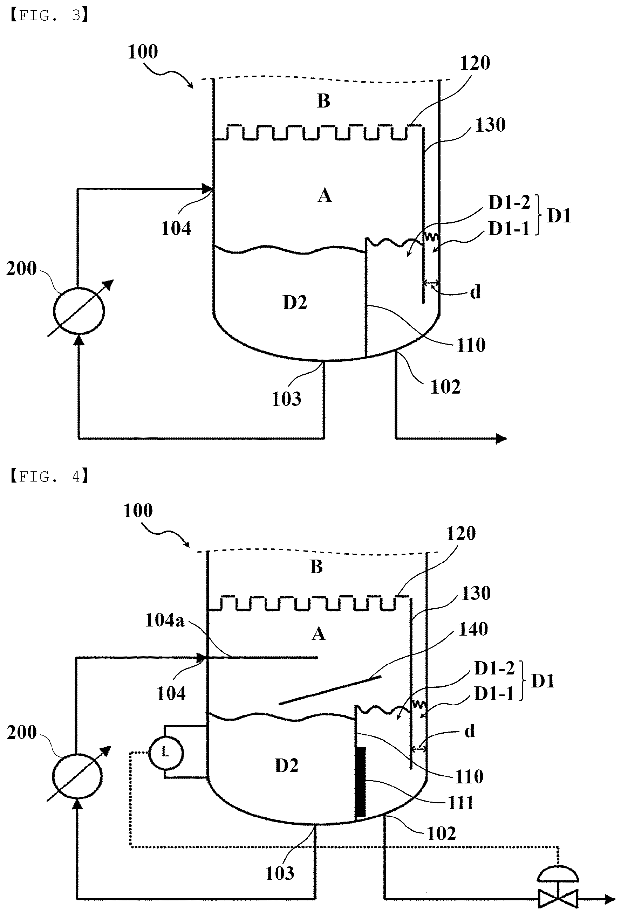

FIG. 2 is a diagram illustrating a refinery apparatus and a process flow according to one embodiment of the present disclosure as an exemplary drawing, and FIGS. 3 and 4 are diagrams illustrating a detailed structure of a bottom region A of a distillation column 100 according to one embodiment of the present disclosure as an exemplary drawing.

Referring to FIG. 2, the refinery apparatus according to the present disclosure includes a distillation column 100. The distillation column 100 is a device capable of separating multi-component substances included in a raw material by their respective boiling point differences. Considering the boiling points of the components of the raw material being introduced or the components to be separated, a distillation column equipped with trays of various shapes may be used in the refinery apparatus of the present disclosure.

According to one embodiment of the present invention, the refinery apparatus is equipped with a reboiler 200 that transfers heat to a bottom discharge stream of the distillation column 100. The reboiler 200 is a heating device separately installed outside the distillation column 100, and may refer to a device for repeatedly heating and evaporating a stream of the material to be refined discharged from the bottom of the distillation column.

If necessary, the refinery apparatus may further be equipped with a condenser (not illustrated) that converts the upper discharge stream of the distillation column 100 into a liquid phase. The condenser is a device separately installed outside the distillation column 100, and refers to a device for cooling a flow discharged from the top of the distillation column by, for example, contacting the flow with cooling water introduced from the outside.

Referring to FIG. 2, the interior of the distillation column 100 is divided into a bottom region A, a middle region B, and a top region C.

In the present specification, the term "bottom region" means a relatively lower part in the structure of the distillation column 100, and for example, can mean the lowermost part among three regions divided in a height direction of the distillation column 100, and more specifically, may mean the lower region of the lowest tray.

In addition, the term "top region" in the present specification means a relatively upper part in the structure of the distillation column 100, and for example, may mean the uppermost part among three regions divided in the height direction of the distillation column 100, and more specifically, may mean the upper region of the uppermost tray.

In addition, in the present specification, the "middle region" means a relatively middle part in the structure of the distillation column 100, and for example, the middle region may mean the middle region among three regions divided in the height direction of the distillation column 100, and more specifically, may mean a region between the bottom region A and the top region C of the distillation column 100, that is, a region between the lowest tray and the uppermost tray.

In the present specification, the bottom region, the top region, and the middle region of the distillation column may be used as relative concepts. The bottom of the distillation column 100 is included in the bottom region, and the top of the distillation column 100 is included in the top region, and unless specifically defined otherwise in the present specification, the bottom region A is used with the same meaning as the lower region, and the top region C is used with the same meaning as the upper region.

In addition, inside the bottom region A of the distillation column 100, a partition wall 110 is provided that is extended to have a predetermined height from the bottom of the distillation column in the sump where the liquid feed is collected, and the sump region is divided into a first region D1 and a second region D2 with the partition wall 110 therebetween. Here, the sump region may mean the bottom region A. In addition, the bottom region A may also be divided into a liquid region and a gaseous region based on the liquid level of the solution.

The height of the partition wall 110 can be appropriately changed depending on the structure or size of the distillation column. However, as illustrated in FIG. 2, the partition wall may be provided so as not to be in contact with a lowest tray 120 to be described later. Here, the predetermined height may mean a height at which the partition wall 110 is spaced so as not to be in contact with the lowest tray 120. In addition, since the upper end of the partition wall 110 is provided so as not to be in contact with the lowest tray 120, the gas in the gas phase region of the bottom region A can move between the first region D1 and the second region D2.

Meanwhile, referring to FIG. 3, the first region D1 and the second region D2 may be partitioned unequally as needed. More specifically, the volume of the first region D1 may be determined according to the flow rate of the stream discharged below the second region D2 and the speed at which the inorganic substance precipitates in the first region D1. In addition, the first region D1 and the second region D2 are partitioned so that the solution accommodated in the first region D1 overflows into the second region D2 and the solution in the second region D2 does not overflow into the first region D1.

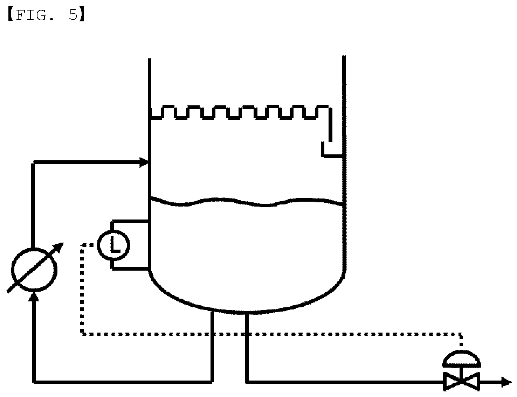

Referring to FIG. 5, conventionally, since there is no partition wall in the distillation column sump, the section where the feed which is the material to be refined is introduced into the sump and the lower discharge stream (reboiler section) circulated to the reboiler for gas-liquid separation are not separated from each other, and thus, inorganic substances are included in the lower discharge stream circulated to the reboiler and passed through the reboiler. In this way, when the inorganic substance enters the reboiler, the inorganic substance droplets are entrained to the gaseous shape of the reflux stream of the gas-liquid mixture generated as the inorganic substances pass through the reboiler and moves to the upper part, which has the problem of lowering the purity of the refined product.

In the present disclosure, in order to solve such a problem, by providing the partition wall 110 in the bottom region A of the distillation column 100 to divide the sump into the first region D1, which is a feed inflow zone and an inorganic substance removal zone, and the second region D2, which is a reboiler zone, it is possible to provide a device which can minimize the amount of inorganic substances introduced into the bottom discharge stream circulated to the reboiler and thus refine a material to be refined with high purity. In addition, a solution inside the second region D2 generates a vertical mixed flow due to the bottom discharge stream of the second region D2 and the liquid reflux stream introduced into the second region D2, but the solution inside the first region D1 is not affected by the flow of the solution inside the second region D2 due to the partition wall 110 positioned between the first region D1 and the second region D2, and can have a gentle flow, and thus, it is possible to precipitate the inorganic substances having a relatively high density in the bottom of the first region D1. In this case, the precipitated inorganic substance can be discharged together with the high-boiling-point organic substance through a first outlet 102 described later.

In the refinery apparatus according to the embodiment of the present invention, referring to FIG. 2, a feed supply port 101 is provided in the distillation column 100, and further, the first outlet 102, a second outlet 103, and a reflux inlet 104 is provided in the lower region of the distillation column, and a third outlet 105 can be further provided in the upper region of the distillation column.

The feed supply port 101 is provided on one side of the distillation column 100. The feed stream including a material to be refined may be supplied to the distillation column 100 through the feed supply port 101. In FIG. 2, the feed supply port 101 is illustrated as being positioned in the middle region B of the distillation column, but is not limited thereto, and a height (number of stages) of the feed supply port may be appropriately selected depending on the composition, temperature, and pressure of the feed. For example, the energy usage of the reboiler varies depending on the composition, temperature, and pressure of the feed, and the height of the feed supply port 101 may be determined accordingly.

The material to be refined may be a commercially available material or be manufactured by a method commonly used in the field. In this case, the material to be refined may include an inorganic substance having an impurity of more than 0 ppb and equal to or less than 100 ppb. For example, the inorganic substance may include one or more metal components selected from among aluminum (Al), arsenic (As), iron (Fe), boron (B), chromium (Cr), cadmium (Cd), titanium (Ti), zinc (Zn), and magnesium (Mg). The type and content of the impurity included in the material to be refined may vary depending on various environments accompanying the reaction and refinery processes.

In one embodiment, when the material to be refined is a crude isopropyl alcohol product, referring to FIG. 1, in a reaction unit 1, a propylene monomer and water are reacted to obtain a reaction product including isopropyl alcohol (IPA), unreacted propylene monomer, unreacted water, n-propyl alcohol (NPA), and by-products such as organic substances, the reaction product is transferred to the gas refinery unit 2 including an absorption column, a gas refinery column, or the like to separate low-boiling-point gas components including unreacted propylene monomer. The reaction product (including IPA, NPA, water, and organic substances) from which the gas components are separated is supplied to an IPA refinery unit 3, and the crude isopropyl alcohol can be obtained by removing organic substances, NPA, and water through a refinery process performed in a plurality of distillation columns.

The crude isopropyl alcohol may be obtained by supplying the reaction product of propylene and water to the gas refinery unit and the IPA refinery unit to separate gas components, organic substances, or the like, but may still include trace amounts of organic substances and water, and in particular, may include inorganic impurities including metal components such as Al, As, Fe, B, Cr, Cd, Ti, Zn, and Mg due to the use of a catalyst during the reaction.

For example, the crude isopropyl alcohol may include 0.08 wt% or less of low-boiling-point organic substances, 0.05 wt% or less of high-boiling-point organic substances, and 100 ppb or less of inorganic substances as impurities based on the total weight thereof. The low-boiling-point organic substances may include, for example, inert gases having C₃ or less, and acetone, and the high-boiling-point organic substances may include, for example, n-propyl alcohol and hexanol, and the inorganic substances may include, for example, metal components such as Al, As, Fe, B, Cr, Cd, Ti, Zn, and Mg, but are not limited thereto.

In order to remove the inorganic substances included in the material to be refined, the present disclosure provides the refinery apparatus capable of recovering a highly refined product by improving the structure of the bottom region A of the distillation column. The distillation column that can be used in the present disclosure may have the same structure as that generally used in petrochemical processes, except for the structure of the bottom region, without any special restrictions.

Meanwhile, the middle region B of the distillation column 100 is provided with a plurality of trays in the form of a perforated plate or grid, and a plurality of stages are divided by the plurality of trays. The plurality of trays are arranged in a vertical direction with respect to the height direction of the distillation column and are arranged so as to be spaced apart from each other. In each stage, a gaseous stream rising upward and a liquid stream falling downward contact each other to transfer heat and mass, and as a result, a part of the high-boiling-point component is condensed and flows down to the bottom, and the uncondensed vapor continues to rise to the top, which is continuously performed.

The number, type, and size of the plurality of trays are not particularly limited, and may be set based on the number of theoretical stages, or the like, inferred from a distillation curve considering the composition of the feed stream. In the above, the "number of theoretical stages" means the number of stages divided by a virtual region or a plurality of trays in which two phases, such as gas and liquid, are in equilibrium with each other in the distillation column 100. For example, a distillation column having the number of theoretical stages of 40 to 60 may be used as the distillation column 100.

Meanwhile, in the refinery apparatus according to one embodiment of the present disclosure, one end of the lowest tray 120, which is a collector tray among the plurality of trays, is positioned above the first region D1, and a guide baffle 130 extending downward from one end of the lowest tray 120 to be adjacent to the bottom of a distillation column is provided. One end of the lowest tray 120 is open so as not to come into contact with the side of the distillation column 100 on the first region D1. Here, a descending liquid stream among the feed streams including the material to be refined is introduced into the first region D1 through the guide baffle 130.

In addition, the lower end of the guide baffle 130 is positioned below the upper end of the partition wall 110, that is, the liquid level of the solution in the first region D1. In addition, as illustrated in FIGS. 3 and 4, the first region D1 may be divided into a 1-1 region D1-1 and a 1-2 region D1-2 by the guide baffle 130.

Referring to FIG. 5, conventionally, the lower end of the guide baffle is positioned above the liquid level of the solution in the sump, the state where the inorganic substance is mixed in the dropped solution is maintained, the flow of the solution in the sump is active, and thus, the inorganic substance could not be precipitated. Meanwhile, in the present disclosure, the lower end of the guide baffle 130 is positioned below the liquid level of the solution in the first region D1, precipitation of the inorganic substance having a relatively high density is induced, and the 1-1 region D1-1 into which the descending liquid stream of the feed stream flows and the 1-2 region D1-2 where the inorganic substance in the solution is precipitated can be separated.

Specifically, after the descending liquid stream from the feed stream flows into the 1-1 region D1-1, the flow that moves to the 1-2 region D1-2 through the space separated between the bottom of the distillation column and the guide baffle is generated. In this case, the solution in the 1-2 region D1-2 can maintain a gentle flow by minimizing the influence of the solution flow dropped into the 1-1 region D1-1 by the guide baffle 130, and accordingly, precipitation of an inorganic substance having a relatively high density can be generated in the 1-2 region D1-2. Furthermore, the precipitated inorganic substance is discharged as a lower discharge stream of the first region, and when the liquid stream flows in such a way that the liquid stream exceeds the internal capacity of the first region D1, the solution in the upper part of the 1-2 region D1-2 where the inorganic substance is minimized moves to the second region D2. Accordingly, the inorganic substance in the lower discharge stream of the second region circulated to the reboiler 200 is minimized, and the inorganic substance droplets entrained to the gaseous reflux stream passing through the reboiler are minimized, so that the purity of the refined product can be further improved.

More specifically, the height h from the bottom of the distillation column to the lower end of the guide baffle satisfies the following Equation (1).$0.5 d < h < 2 d$

Here, d is a maximum gap opened between one end of the lowest tray and the side of the distillation column.

When the height h from the bottom of the distillation column to the lower end of the guide baffle satisfies the range of the Equation (1), the influence of the flow of the solution in the 1-2 region D1-2 by the flow moving from the 1-1 region D1-1 to the 1-2 region D1-2 can be minimized. That is, the flow moving from the 1-1 region D1-1 to the 1-2 region D1-2 can be maintained in a gentle state, thereby suppressing the mixing flow of the solution in the 1-2 region D1-2 and inducing the precipitation of inorganic substances.

Meanwhile, referring to FIG. 4, a magnetic bar 111 for removing an inorganic substance may be further provided on at least one side of the partition wall 110. When the magnetic bar 111 is provided, the removal efficiency of an inorganic substance having magnetism, such as iron (Fe) and magnetite, included in the solution can be improved. As described above, since a relatively high-density inorganic substance is precipitated in the first region D1, it may be preferable for the magnetic bar 111 to be positioned in the first region D1 in terms of the inorganic substance removal efficiency, but it is not limited thereto. For example, when the magnetic bar 111 is positioned in the first region D1, an inorganic substance having magnetism may be attached to the magnetic bar or guided to the lower part of the 1-2 region D1-2, making it easier to discharge through the first outlet 102 described later.

The first outlet 102 is positioned at the bottom of the first region D1 and may discharge the lower discharge stream of the first region D1 including the high-boiling-point organic substances and inorganic substances. A descending liquid stream among the feed stream including the material to be refined flows into the first region D1 through the guide baffle 130, and the solution flowing into the first region D1 includes the high-boiling-point organic substances and inorganic substances. Some of the solution introduced into the first region D1 is discharged as a lower discharge stream of the first region D1 through the first outlet 102, and the remaining solution exceeding the internal capacity of the first region D1 flows into the second region D2. Here, a gentle solution flow is formed inside the 1-2 region D1-2, inorganic substances are precipitated downward, and accordingly, most of the inorganic substances may be included in the lower discharge stream of the first region D1 and discharged. More specifically, since more inorganic substances are precipitated, especially, in the lower part of the 1-2 region D1-2 by the guide baffle 130, the inorganic substance removal efficiency can be further improved when the first outlet 102 is positioned at the lower part of the 1-2 region D1-2.

For example, the high-boiling-point organic substances discharged through the first outlet 102 may include n-propyl alcohol, hexanol, or a mixture thereof, and the inorganic substance may include one or more metal components selected from Al, As, Fe, B, Cr, Cd, Ti, Zn, and Mg.

Meanwhile, as illustrated in FIG.4, the flow rate of the lower discharge stream of the first region D1 may be controlled using a level control valve, and specifically, the flow rate of the lower discharge stream of the first region D1 discharged through the first outlet 102 may be controlled according to the water level of the solution in the second region D2.

The second outlet 103 is provided at the bottom of the second region D2 and is connected to one side of the reboiler 200. The solution of the first region D1 flows into the second region D2 over the partition wall 110, and the solution in the second region D2 is discharged through the second outlet 103, passes through the reboiler 200, and is then recycled through the reflux inlet 104 described later and flows back into the second region D2. In this case, the solution flowing from the first region D1 into the second region D2 may be in a state where most of the inorganic substances have been removed.

The reflux inlet 104 is positioned at the upper part of the second region D2 and is connected to the other side of the reboiler 200. More specifically, the reflux inlet 104 can be positioned at a height between the lowest tray 120 and the liquid level of the solution in the second region D2. The gas-liquid mixed phase stream that has passed through the reboiler 200 through the reflux inlet 104 may be refluxed to the bottom region A of the distillation column. The liquid in the refluxed gas-liquid mixed phase stream moves downward and is received in the second region D2, and the vapor moves to the upper middle region B and undergoes a gas-liquid separation process.

The reflux inlet 104 may be further provided with a pipe 104a extending inwardly of the distillation column. The extension pipe 104a may be provided in a horizontal direction. The flow of the liquid phase moving downward among the gas-liquid mixed phase stream refluxed through the extension pipe 104a is induced to descend adjacent to a slope baffle 140 described later, thereby preventing the flow of the liquid phase from being mixed by passing over to the first region D1, and the flow of the liquid phase of the refluxed gas-liquid mixed phase stream can be induced to gently flow to the second region D2.

As illustrated in FIG. 4, the slope baffle 140 may be further provided in the bottom region A of the distillation column 100 as needed. The reflux stream introduced through the reflux inlet 104 has a high flow rate and a high flow velocity, so that a part of the reflux stream may flow over to the first region D1 and affect the flow of the solution within the first region D1, which may hinder the gentle flow of the solution within the first region D1 and prevent the precipitation of inorganic substances. In addition, the reflux stream may be mixed with the solution introduced from the lowest tray (that is, collector tray) 120, so that inorganic substances may flow into the second region D2. In this case, when the slope baffle 140 is provided, the reflux stream can be prevented from mixing with the solution flowing in from the lowest tray 120 or the solution in the first region D1. In addition, the descending liquid phase flow of the refluxed gas-liquid mixed phase stream may be induced to flow gently to the second region D2 by passing through the slope baffle 140.

In detail, the slope baffle 140 may be provided to be positioned at a height between the reflux inlet 104 and the upper end of the partition wall 110. More specifically, one end of the slope baffle 140 may be positioned above the first region D1, and the other end of the slope baffle 140 may be positioned above the second region D2. In addition, it is preferable that the other end of the slope baffle positioned above the second region D2 be positioned at a height lower than one end of the slope baffle positioned above the first region D1 so that the solution coming into contact with the slope baffle 140 can be guided to the second region D2. For example, the inclination of the slope baffle 140 may be 5° to 30°, specifically, 10° to 25°, more specifically, 15°to 20°. Here, the slop means the angle of slop with respect to the horizontal floor.

According to one embodiment, a third outlet 105 may be positioned in the top region C of the distillation column. For example, when the material to be refined is crude isopropyl alcohol, isopropyl alcohol (IPA) and/or low-boiling-point organic substances included in the crude isopropyl alcohol may be discharged through the third outlet 105. The low-boiling-point organic substances may include an inert gas having a temperature of C₃ or lower, acetone, or a mixture thereof.

In addition, a part of the stream discharged through the third outlet 105 may be discharged through a condenser (not illustrated) and the remainder may be converted into a liquid phase and refluxed back to the distillation column 100 through an inlet 106 provided in the top region C.

For example, the material to be refined supplied to the distillation column 100 undergoes a separation process by continuous gas-liquid contact in each stage included in the middle region B, and relatively low-boiling-point organic substances and products (for example, isopropyl alcohol) rise in a vapor state and are discharged through the third outlet 105 of the top region C, and relatively heavy components, such as high-boiling-point organic substances and inorganic substances, may descend in a condensed state and flow out through the first outlet 102 of the bottom region A. In addition, the effluent of the top region C may include a trace amount of water included in the material to be refined. Furthermore, the effluent of the top region C may undergo an additional refinery process as needed, through which a high-purity product may be ultimately obtained.

Hereinabove, the refinery apparatus according to the present invention has been described and illustrated in the drawings, but the description and illustration of the drawings describe and illustrate only the core configuration for understanding the present invention, and in addition to the processes and devices described and illustrated in the drawings, processes and devices not described and illustrated separately can be appropriately applied and utilized to implement the refinery apparatus according to the present disclosure.

### [Description of Reference Signs]

100: distillation column, 200: reboiler
A: bottom region, B: middle region, C: top region
D1: first region, D2: second region
D1-1: 1-1 region, D1-2: 1-2 region
101: feed supply port, 102: first outlet
103: second outlet, 104: reflux inlet, 104a: extension pipe
110: partition wall, 111: magnetic bar
120: lowest tray, 130 guide baffle, 140: slope baffle

## Claims

1. A refinery apparatus, comprising:
a distillation column (100); and
a reboiler (200),
wherein an interior of the distillation column (100) is divided into a bottom region (A), a middle region (B), and a top region (C),
the bottom region (A) comprises a first region (D1) and a second region (D2) separated by a partition wall (110) extending from a bottom of the distillation column (100) to have a predetermined height therebetween,
the distillation column (100) comprises:
a feed supply port (101) provided at one side of the middle region (B) to supply a feed stream including a material to be refined to the distillation column (100),
a first outlet (102) provided below the first region (D1),
a second outlet (103) provided below the second region (D2) and connected to one side of the reboiler (200),
a reflux inlet (104) provided above the second region (D2) and connected to the other side of the reboiler (200), and
a plurality of trays arranged vertically apart from the partition wall (110) provided in the middle region (B) of the distillation column (100),
wherein one end of a lowest tray (120) among the plurality of trays is opened so as not to contact a side of the distillation column (100) on the first region (D1), and a guide baffle (130) extending downward from one end of the lowest tray (120) is provided,
wherein a height h from the bottom of the distillation column (100) to a lower end of the guide baffle (130) satisfies the following Equation 1:
$0,5 d < h < 2 d$
wherein, d is a maximum gap opening between one end of the lowest tray (120) and the side of the distillation column (100).

2. The refinery apparatus of claim 1,
wherein the reflux inlet (104) further comprises a pipe (104a) extending inwardly of the distillation column (100).

3. The refinery apparatus of claim 1, further comprising a slope baffle (140) provided at a height between the reflux inlet (104) and an upper end of the partition wall (110).

4. The refinery apparatus of claim 3,
wherein one end of the slope baffle (140) is positioned above the first region (D1), and
the other end of the slope baffle (140) is positioned above the second region (D2).

5. The refinery apparatus of claim 3,
wherein the slope baffle (140) is provided such that the other end above the second region (D2) is positioned at a height lower than the one end above the first region (D1).

6. The refinery apparatus of claim 1, further comprising a magnetic bar (111) provided on at least one side of the partition wall (110).

7. The refinery apparatus of claim 6,
wherein the magnetic bar (111) is positioned in the first region (D1).

## Patentansprüche

1. Raffinerievorrichtung, die umfasst:
eine Destillationskolonne (100); und
einen Verdampfer (200),
wobei ein Inneres der Destillationskolonne (100) in einen unteren Bereich (A), einen mittleren Bereich (B) und einen oberen Bereich (C) unterteilt ist,
der untere Bereich (A) einen ersten Bereich (D1) und einen zweiten Bereich (D2) umfasst, die durch eine Trennwand (110) getrennt sind, die sich von einem Boden der Destillationskolonne (100) erstreckt, um eine vorbestimmte Höhe dazwischen aufzuweisen,
wobei die Destillationskolonne (100) umfasst:
eine Zufuhröffnung (101), die an einer Seite des mittleren Bereichs (B) vorgesehen ist, um der Destillationskolonne (100) einen Zufuhrstrom zuzuführen, der ein zu raffinierendes Material enthält,
einen ersten Auslass (102), der unter dem ersten Bereich (D1) vorgesehen ist,
einen zweiten Auslass (103), der unter dem zweiten Bereich (D2) vorgesehen ist und mit einer Seite des Verdampfers (200) verbunden ist,
einen Rückflusseinlass (104), der über dem zweiten Bereich (D2) vorgesehen ist und mit der anderen Seite des Verdampfers (200) verbunden ist, und
eine Vielzahl von Böden, die vertikal getrennt von der Trennwand (110) angeordnet sind, die in dem mittleren Bereich (B) der Destillationskolonne (100) vorgesehen ist,
wobei ein Ende eines untersten Bodens (120) unter der Vielzahl von Böden geöffnet ist, um eine Seite der Destillationskolonne (100) auf dem ersten Bereich (D1) nicht zu berühren, und eine Leitplatte (130), die sich von einem Ende des untersten Bodens (120) nach unten erstreckt, vorgesehen ist,
wobei eine Höhe h von dem Boden der Destillationskolonne (100) zu einem unteren Ende der Leitplatte (130) die folgende Gleichung 1 erfüllt:
$0,5 d < h < 2 d$
wobei d eine maximale Spaltöffnung zwischen einem Ende des untersten Bodens (120) und der Seite der Destillationskolonne (100) ist.

2. Raffinerievorrichtung nach Anspruch 1,
wobei der Rückflusseinlass (104) ferner ein Rohr (104a) umfasst, das sich von der Destillationskolonne (100) nach innen erstreckt.

3. Raffinerievorrichtung nach Anspruch 1, die ferner eine Neigungsplatte (140) umfasst, die in einer Höhe zwischen dem Rückflusseinlass (104) und einem oberen Ende der Trennwand (110) vorgesehen ist.

4. Raffinerievorrichtung nach Anspruch 3,
wobei ein Ende der Neigungsplatte (140) über dem ersten Bereich (D1) positioniert ist, und
das andere Ende der Neigungsplatte (140) über dem zweiten Bereich (D2) positioniert ist.

5. Raffinerievorrichtung nach Anspruch 3,
wobei die Neigungsplatte (140) derart vorgesehen ist, dass das andere Ende über dem zweiten Bereich (D2) in einer niedrigeren Höhe als das eine Ende über dem ersten Bereich (D1) positioniert ist.

6. Raffinerievorrichtung nach Anspruch 1, die ferner einen Magnetstab (111) umfasst, der auf mindestens einer Seite der Trennwand (110) vorgesehen ist.

7. Raffinerievorrichtung nach Anspruch 6,
wobei der Magnetstab (111) in dem ersten Bereich (D1) positioniert ist.

## Revendications

1. Appareil de raffinerie, comprenant :
une colonne de distillation (100) ; et
un rebouilleur (200),
l'intérieur de la colonne de distillation (100) se composant d'une zone inférieure (A), d'une zone intermédiaire (B) et d'une zone supérieure (C),
la zone inférieure (A) comprenant une première zone (D1) et une deuxième zone (D2), séparées par une cloison (110) s'étendant du bas de la colonne de distillation (100), afin de disposer d'une hauteur prédéterminée entre elles,
la colonne de distillation (100) comprenant:
un orifice d'approvisionnement (101) agencé sur un côté de la zone intermédiaire (B) pour introduire un flux d'alimentation comprenant une matière à raffiner dans la colonne de distillation (100),
une première sortie (102) agencée sous la première zone (D1),
une deuxième sortie (103) agencée sous la deuxième zone (D2) et reliée à un côté du rebouilleur (200),
un orifice d'entrée du reflux (104) agencé au-dessus de la deuxième zone (D2) et relié à l'autre côté du rebouilleur (200), et
une pluralité de plateaux disposés verticalement à l'écart de la cloison (110) située dans la zone intermédiaire (B) de la colonne de distillation (100),
un bout d'un plateau inférieur (120) parmi la pluralité des plateaux étant ouvert de manière à ne pas se trouver au contact d'un côté de la colonne de distillation (100) sur la première zone (D1), et une grille de guidage (130) s'étendant vers le bas d'un bout du plateau inférieur (120) étant agencée,
une hauteur h du bas de la colonne de distillation (100) jusqu'à une extrémité inférieure de la grille de guidage (130) répondant à l'équation 1 suivante : $0 , 5 d < h < 2 d$ d étant une ouverture maximale entre un bout du plateau inférieur (120) et le côté de la colonne de distillation (100).

2. Appareil de raffinerie selon la revendication 1,
l'orifice d'entrée du reflux (104) comprenant en outre un tuyau (104a) s'étendant de la colonne de distillation (100) vers l'intérieur.

3. Appareil de raffinerie selon la revendication 1, comprenant en outre un déflecteur incliné (140) agencé à une hauteur entre l'orifice d'entrée du reflux (104) et une extrémité supérieure de la cloison (110).

4. Appareil de raffinerie selon la revendication 3,
un bout du déflecteur incliné (140) étant positionné au-dessus de la première zone (D1), et
l'autre bout du déflecteur incliné (140) étant positionné au-dessus de la deuxième zone (D2).

5. Appareil de raffinerie selon la revendication 3,
le déflecteur incliné (140) étant agencé de sorte que l'autre bout au-dessus de la deuxième zone (D2) soit positionné à une hauteur inférieure à celle du bout au-dessus de la première zone (D1).

6. Appareil de raffinerie selon la revendication 1, comprenant en outre une barre magnétique (111) agencée sur au moins un côté de la cloison (110).

7. Appareil de raffinerie selon la revendication 6,
la barre magnétique (111) étant positionnée dans la première zone (D1).
